Europäisches Patentamt

European Patent Office

Office européen des brevets

(19)

(11) **EP 0 580 736 B1**

(12) # EUROPEAN PATENT SPECIFICATION

(45) Date of publication and mention
of the grant of the patent:
**09.04.1997 Bulletin 1997/15**

(21) Application number: **92910378.6**

(22) Date of filing: **24.03.1992**

(51) Int Cl.[6]: **C07C 7/148**, C08F 110/06

(86) International application number:
**PCT/US92/02392**

(87) International publication number:
**WO 92/17428 (15.10.1992 Gazette 1992/26)**

(54) **PROCESS FOR UPGRADING THE QUALITY OF LIGHT ENDS**

VERFAHREN ZUR VERBESSERUNG DER QUALITÄT VON LEICHTFRAKTIONEN

PROCEDE PERMETTANT D'AMELIORER LA QUALITE DES FRACTIONS LEGERES

(84) Designated Contracting States:
**BE DE ES FR GB IT NL SE**

(30) Priority: **08.04.1991 US 682040**

(43) Date of publication of application:
**02.02.1994 Bulletin 1994/05**

(73) Proprietor: **EXXON CHEMICAL PATENTS INC.
Linden, New Jersey 07036-0710 (US)**

(72) Inventors:
• **VENKATRAM, Ramdas
Morris Township, NJ 07960 (US)**

• **MILLIMAN, George, Elmer
Fanwood, NJ 07023 (US)**

(74) Representative: **Bawden, Peter Charles et al
EXXON CHEMICAL LIMITED
Exxon Chemical Technology Centre
PO Box 1
Abingdon Oxfordshire OX13 6BB (GB)**

(56) References cited:
**EP-A- 0 111 911        EP-A- 0 379 394
BE-A- 901 355           FR-A- 2 145 688
FR-A- 2 444 653         US-A- 4 433 981
US-A- 4 835 338**

## Description

### 1. Field of the Invention

The present invention relates to processes for removing $AsH_3$ or $PH_3$ impurities from hydrocarbon streams. More particularly, the present invention is directed to removing $AsH_3$ or $PH_3$ impurities from hydrocarbon streams by contacting the hydrocarbon stream with a chemical absorbent including a modified alumina having a surface area within the range of about 10 $m^2/$g to about 300 $m^2/g$ and a pore volume within the range of about 0.1 mL/g to about 1 mL/g so as to result in a purified hydrocarbon stream containing a reduced amount of the impurities.

Specifically, the present invention is directed to removing $AsH_3$ or $PH_3$ impurities from light ends, such as propylene, by contacting the propylene containing an initial amount of such an impurity with a chemical absorbent comprising a modified alumina having a surface area within a range of about 10 $m^2/g$ to about 300 $m^2/g$ and a pore volume within the range of about 0.1 mL/g to about 1 mL/g under contacting conditions effective to result in a purified propylene stream containing a reduced amount of such impurities.

### 2. Discussion of Background and Material Information

Impurities present in hydrocarbon streams, and particularly from light ends, such as propylene, pose various problems including contamination and of downstream processes, such as propylene polymerization adversely impacting catalyst utilization efficiency and product quality.

Conventionally, methods have been proposed to remove certain impurities, such as carbon dioxide, from gaseous streams which involve the use of liquids, such as solutions containing ethanolamine, ammonia, soda, carbonates and lyes. These methods are ineffective in reducing the impurities to the extremely low levels required for the latest generation of highly selective catalysts in the downstream processes, such as propylene polymerization.

U.S. Patent No. 3,141,729 discloses another approach which involves the use of solid adsorbent materials, such as molecular sieves, calcium oxide, finely-divided micro-porous silver oxide dispersed in an admixture with aluminum oxide, and supported cogels of divalent and trivalent metals for such purpose.

U.S. Patent No. 3,865,924 discloses the use of a synergistic mixture of carbonate and alumina for similar purposes, but requires the addition of water since the adsorption reaction is:

$$K_2CO_3 + CO_2 + H_2O \rightarrow 2KHCO_3.$$

U.S. Patent No. 4,433,981, SLAUGH at al., is directed to the removal of carbon dioxide from a gaseous stream by contacting the stream with an adsorbent prepared by impregnating a porous alumina with an alkali metal or alkaline earth metal oxide or salt decomposable upon calcination, and subsequently calcining the impregnated alumina at about 350°C - 700°C.

In general, alumina is a known adsorbent in many chemical processes, such as the polymerization of olefins, such as ethylene, for the removal of water and small concentrations of methanol, carbonyl-containing compounds and peroxides. However, the use of alumina has certain disadvantages which adversely affect its use as an adsorbent. Among such disadvantages is that alumina is not always effective as an adsorbent for the removal of, for example, $CO_2$, from gaseous olefin-containing streams which contain $CO_2$ at low level concentrations, for example, down to 1 ppm.

Molecular sieves have been used as adsorbents for $CO_2$, but in some instances have been found to be inefficient when used for the removal of $CO_2$ from a gaseous stream containing low molecular weight olefins, such as ethylene.

Caustic scrubbers, or bulk caustic scrubbers, have been proposed for use as adsorbents for $CO_2$ from a gaseous stream, but suffer certain disadvantages, including posing safety problems and adding water to the stream.

U.S. Patent No. 4,493,715 is directed to a process for the removal of $CO_2$ from a gaseous stream containing at least one $C_2$ to $C_4$ olefin which involves contacting the stream with a regenerable calcined alkali metal compound-treated alumina.

U.S. Patent 4,614,729, STAUFFER CHEMICAL COMPANY, discloses the removal of catalyst poisons, such as $CO_2$, CO, COS, $H_2O$, $H_2S$, $O_2$, and acetylenes from hydrocarbon fluids, such as liquified $C_2$ - $C_{30}$ alkenes. The catalyst used for this purpose is alumina treated with an organometallic compounds from Groups II, III and IV.

British Patent No. GB 1,383,611, FARBEWERKE HOECHST, discloses the removal of impurities at levels below 10 ppm from propylene by treating compressed propylene with mineral adsorbents, such as alumina, which may also contain other oxides, such as $Na_2O$.

Belgium Patent No. BE 901,355, LABOFINA, is directed to the removal of low level impurities by passing the propylene over alumina treated with trialkyl aluminum. The purified propylene is disclosed as containing less than 30 ppm COS.

U. S. Patent No. 4,798,711, NOXSO CORP., describes a process for removing nitrogen oxides and/or sulfur oxides from a gas by using an alkali metal-alumina sorbent.

Katalco 59-3 has been disclosed as a dechlorination catalyst which is specially formulated for the removal of chlorides in advertising publications, i.e., product bulletin KAT-59-3-1. As described Katalco 59-3 is to be used principally for the removal of reactive chlorides from natural gases and other hydrocarbon streams by absorption; for the removal of organic chlorides following hydrogenation over a hydro-treating catalyst; and the dehalogenation of combined chloride/fluoride hydrocarbon streams. Katalco 59-3 is disclosed as having a high chloride capacity, and reduces the chloride content of gas streams to below the normal detection level. It is also disclosed as being compatible with liquid hydrocarbons and operates over a wide range of process conditions. As advertised in advertising literature designated CL-22-1, Katalco 59-3 is described as a solid chemical adsorbent for the removal of chlorides from gas streams. It is also disclosed as being useful to purify any dry process stream where there is a need to prevent corrosion or filing of downstream equipment or deactivation of process catalysts.

SUMMARY OF THE INVENTION

Notwithstanding the previously-described efforts, catalysts used in the manufacture of polypropylene are extremely sensitive to impurities in the propylene feedstock such as $CO_2$, $COS$, $H_2S$, $H_2O$, arsine, and phosphine. In an attempt to solve the problems associated with catalyst deactivation as a consequence of the presence of such impurities in the propylene feedstock there is a continuing concern to achieve lower and lower levels of these impurities in the propylene feedstock.

Accordingly, the present invention is based on a process for removing $A_sH_3$ or $PH_3$, and optionally $COS$, $H_2O$, $CO_2$ or $H_2S$ impurities, from hydrocarbon streams which comprises contacting a hydrocarbon stream containing an initial amount of $A_sH_3$ or $PH_3$ impurity with a chemical adsorbent comprising a modified alumina having a surface area of from 10 to 300 $m^2/g$ and a pore volume of from 0.1 to 1 ml/g under contacting conditions effective to result in a purified hydrocarbon stream containing a reduced amount of impurities.

The chemical adsorbent which is particularly suitable for purposes of the present invention preferably has a surface area of 60 $m^2/g$ and a pore volume of 0.3 mL/g, and more preferably also has a bulk density within the range of 640.6 to 960.9 $Kg/m^3$ (40 lb./$ft^3$ to about 60 lb./$ft^3$). More preferably, the chemical adsorbent in accordance with the present invention is spherical modified alumina having the previously-described characteristics, which preferably have a size within the range of 0.15875 cm (1/16") diameter to 0.47625 cm (3/16") diameter. The modified alumina chemical adsorbent preferred for purposes of the present invention is impregnated with a metallic substance selected from the group consisting of lithium, potassium, sodium, calcium, magnesium and barium; and the alumina is selected from the group of sources of alumina consisting of alpha alumina and gamma alumina. Most preferably the chemical adsorbent is a modified alumina having a surface area of 60 $m^2/g$, a pore volume of 0.3 mL/g and a bulk density of 880.8 $Kg/m^3$ (55 lb./$ft.^3$) in spherical form having a size within the range of 0.15875 cm (1/16") diameter to 0.47625 cm (3/16") diameter, and is commercially available from Katalco Corporation as Katalco 59-3.

In accordance with the present invention, the impurities which may be removed include at least one selected from the group consisting of $CO_2$, $COS$, $H_2S$ and $H_2O$ in addition to $AsH_3$ or $PH_3$.

In accordance with the present invention, $CO_2$ present in the hydrocarbon stream may be reduced to less than about 1.5% based on the initial amount of the impurity in the hydrocarbon feed stream.

The chemical absorption process of the present invention is performed at contacting conditions which include temperatures within the range of -12.2 to 65.6°C (10°F to 150°F), and preferably within the range of -1.1 to 48.9°C (30°F to 120°F); and pressures within the range of 344.8 to 3447.5 KPag (50 psig to 500 psig), and preferably pressures within the range of 1034.25 to 3413.25 KPag (150 psig to 350 psig); and space velocities within the range of 0.1 VHSV to 25 VHSV, and preferably within the range of 0.5 VHSV to 1 VHSV.

The process of the present invention is preferably performed to remove impurities from hydrocarbon streams in the liquid phase, preferably wherein the hydrocarbon streams include a member selected from the group consisting of olefins, saturated hydrocarbons and mixture thereof. More preferably the light end olefins may be selected from the group of $C_2$ olefins and saturated hydrocarbons, $C_3$ olefins and saturated hydrocarbons, $C_4$ olefins and saturated hydrocarbons, and mixtures of $C_2$ to $C_4$ olefins and saturated hydrocarbons. Most preferably, however, the light ends are selected from the group consisting of ethane, propane, butanes, ethylene, and propylene, and most preferably propylene.

In accordance with the present invention, the modified alumina chemical adsorbent is loaded in adsorbent beds located downstream from conventional alumina driers or in lieu of such conventional alumina driers.

BRIEF DESCRIPTION OF DRAWINGS

Fig. 1 is a graph illustrating the experimental data generated in Example 1 (comparative example) discussed below.

Fig. 2 is another graph of experimental data generated in Example 1.

Fig. 3 is another graph of experimental data generated in Example 1.

Fig. 4 is a flow diagram of the process for upgrading the quality of light ends in accordance with the present invention.

Fig. 5 is a flow diagram of the process of the present invention wherein the modified alumina adsorbent is loaded in adsorbent beds located downstream from alumina driers.

Fig. 6 is a flow diagram of a propylene process, wherein propylene, treated in accordance with the present invention, is used as a feedstock.

DETAILED DESCRIPTION

Impurities, including $AsH_3$ or $PH_3$, and optionally COS, $H_2O$, $CO_2$ or $H_2S$, are effectively removed from hydrocarbon streams, such as liquid olefin streams, using the chemical adsorbents in accordance with the present invention.

In accordance with the present invention, certain chemical adsorbents, have unexpectedly been discovered to be particular effective for the removal of impurities from liquid hydrocarbon streams, such as olefins, for example, propylenes, and are particularly effective for the removal of such impurities from propylene streams.

The chemical adsorbent most preferred for purposes of the present invention is a modified alumina having a surface area within the range of 10 $m^2$/g to 300 $m^2$/g and a pore volume within the range of 0.1 mL/g to 1 mL/g, and more preferably also having a surface area within the range of 60 $m^2$/g and a pore volume of 0.3 mL/g, as well as a bulk density within the range of 640.6 to 960.9 Kg/$m^2$ (40 lb./ft.$^3$ to 60 lb./ft.$^3$), most preferably wherein the bulk density is 880.8 Kg/$m^3$ (55 lb./ft.$^3$), wherein the modified alumina is spherical having a size within the range of 0.16 cm (1/16") diameter to 0.48 cm (3/16") diameter, and is prepared, for example, by impregnating or providing an alumina with a metal selected from the group consisting of lithium, potassium, calcium, magnesium, barium and sodium.

The most preferred adsorbent for purpose of the present invention is a solid chemical adsorbent commercially marketed as Katalco 59-3 by Katalco, a business unit of ICI Americas, Inc.

In accordance with the present invention, such chemical adsorbents have been found to be extremely effective in removing relatively small amounts of such impurities to produce a propylene stream which is essentially devoid of such impurities, i.e., which are present in the resultant purified hydrocarbon stream at less than 1.5%, and more preferably less than 10 ppb.

This is particularly unexpected inasmuch as Katalco 59-3 has been disclosed as a dechlorination catalyst which is specially formulated for the removal of chlorides in advertising publications. In this regard, Katalco 59-3 has been described as being used principally for: the removal of reactive chlorides from natural gases and other hydrocarbon streams by absorption; for the removal of organic chlorides following hydrogenation over a hydro-treating catalyst; and the dehalogenation of combined chloride/fluoride hydrocarbon streams. Katalco 59-3 is disclosed as having a high chloride capacity, and reduces the chloride content of gas streams to below the normal detection level. Katalco 59-3 is further described as a solid chemical adsorbent for the removal of chlorides from gas streams. It is also disclosed as being useful to purify any dry process stream where there is a need to prevent corrosion or filing of downstream equipment or deactivation of process catalysts. Katalco 59-3 has also been disclosed as being useful to purify catalytic reformer off-gases containing ammonium chloride and HCl so as to prevent fouling problems associated with these chlorides. Katalco 59-3 is described as a high capacity adsorbent exhibiting 5-6 times higher absorptive capacity than alternative products, such as activated alumina; in this regard, Katalco 59-3 is disclosed as absorbing up to 3.86 Kg (8.5 lb.) of chloride per 0.28 $m^3$ (ft.$^3$). Katalco 59-3 has been described as having a unique pore structure and chemically-active surface area which provides rapid chloride pickup, chloride absorption close to 100% theoretical in the saturated zone, and no chloride slip. Notwithstanding such disclosures, it is not believed that, prior to the present invention, KATALCO 59-3 was used in a manner disclosed and claimed herein for purposes of the present invention.

Suitable impregnating metal compounds for purposes of the present invention include lithium, potassium, sodium, calcium, magnesium, and barium.

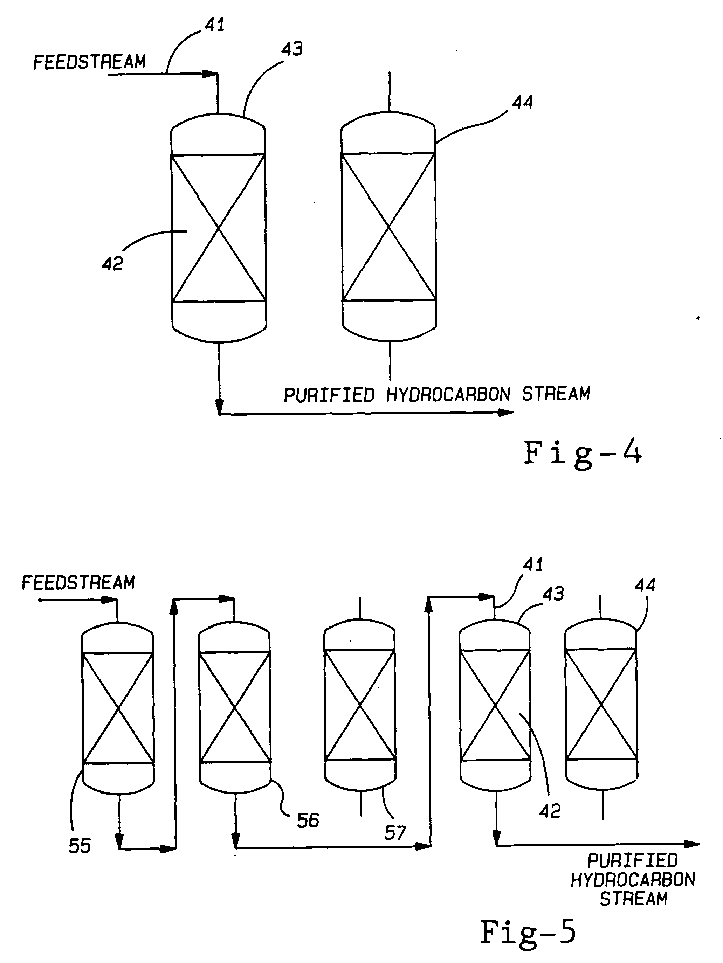

The process for removing impurities from hydrocarbon streams using the chemical adsorbent in accordance with the present invention, referring to Fig. 4, involves contacting the hydrocarbon stream 41 containing saturated hydrocarbons and/or olefins, including an initial amount of an impurity with the chemical adsorbent 42 under contacting conditions effective to result in a purified olefin stream containing a reduced amount of the impurity. The chemical adsorbent is preferably contained in an adsorbent bed 43, zone or other suitable vessel and the hydrocarbon stream, preferably containing olefins, is fed to the chemical adsorbent bed under conditions suitable for the adsorbent bed and process system. Optionally, at least one other adsorbent bed 44, which is essentially the same in structure and adsorbent content, may be provided to alternate with adsorbent bed 43 if taken off-line. Suitable contacting conditions include a pressure within the range of 344.8 to 3447.5 kPag (50 to 500 psig), and preferably (1034.25 to 3413.25 kPa (150-350 psig); temperatures within the range of -12.2 to 65.6°C (10°F to 150°F), and preferably within the range of -1.1 to 18.9°C (30°F to 120°F); and a space velocity within the range of 0.1 to 25 VHSV and preferably within the range of 1 to 15 VHSV. The olefin stream, most preferably in the liquid phase, is fed to the chemical adsorbent bed at 5 VHSV at a temperature of 15.5°C (60°C) under a pressure of 2068.5 KPag (300 psig).

Referring now to Fig. 5, a process for removing impurities using the chemical adsorbent in accordance with the present invention is shown in a flow diagram which illustrates an embodiment where the modified alumina adsorbent 42 is loaded in adsorbent beds 43,44 located downstream from at least one alumina drier, but preferably two alumina driers 55, 56. Optionally, a spare alumina drier 57 may be provided to alternate with either or both of alumina driers 55,56 if one or both is/are taken off-line.

Although for purposes of the present invention the examples have been conducted using the olefin in the gaseous phase, it is believed that the reagent would be equally or more effective, when the hydrocarbon stream is a liquid rather than a gas. Moreover although the present invention has been described for treating an olefin stream, such as propylene, feeds such as ethylene may also be treated in accordance with the present invention, for the reason that, as shown in the examples, the reagent is effective in removing impurities from gaseous streams such as Helium and Propylene. As indicated above, the reagent is expected to be equally effective, if not more, for removal of these impurities from liquid hydrocarbon streams. The reagent, therefore, is suitable for removing impurities from both liquid and gaseous hydrocarbon streams.

Therefore, in general, the process of the present invention may be carried out at a temperature of -12.2 to 65.6°C (10°F to 150°F). The preferred temperatures which have been used for purposes of the present invention are within the range of -1.1 to 48.9°C (30°F to about 120°F).

Similarly, pressures under which the chemical absorption process of the present invention may be practiced will be from 344.8 to 3447.5 KPag (50 psig to 500 psig). The pressures which have been used and are preferred for purposes of the present invention fall within the range of 1034.25 to 3413.25 KPag (150 psig to 350 psig).

The following is a detailed description of the chemical absorption process in accordance with the present invention wherein a propylene stream containing impurities was contacted with the chemical adsorbent comprising a modified alumina in accordance with the present invention, so as to reduce the initial amount of the impurities.

The light ends stream processed was polymer grade propylene. The normal analysis of the stream is as follows: Propylene: >99.5 %; Propane: < 0.5 %; $CO_2$: <2 wppm; COS : <1 wppm; $AsH_3$: < 200 wppb; $H_2O$: < 10 wppm. Experiments conducted with $CO_2$ and $H_2S$ (in Helium carrier gas) have shown that the Katalco reagent would almost quantitatively remove these impurities i.e. to below detectable levels using current measuring techniques. A significant amount of COS and $AsH_3$, are also removed by Katalco 59-3.

The purified hydrocarbon stream, i.e., light ends, and most preferably propylene, which has a substantially reduced amount of impurities may then be processed, for example, in polymerization processes to produce polypropylene wherein the polymerization catalysts, which are otherwise extremely sensitive to the impurities which would otherwise be present in the propylene feedstock, remain active for substantially longer periods of time than if the propylene stream was not treated in accordance with the present invention.

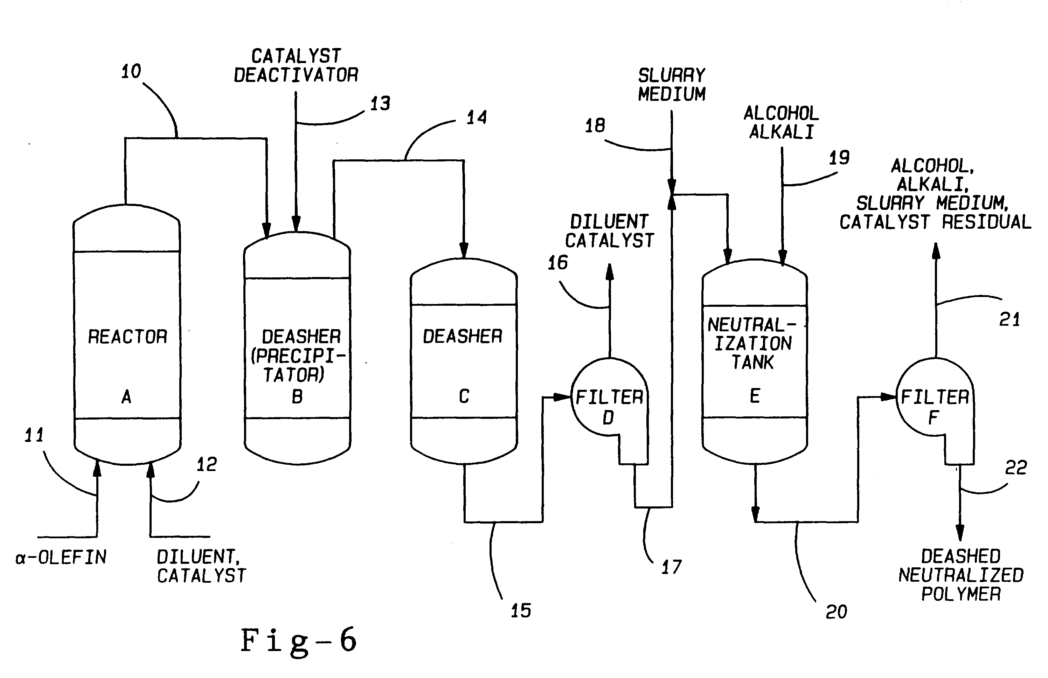

Reference is made to Figure 6 which schematically illustrate a propylene process wherein propylene, purified in accordance with the present invention is used as a feedstock. The attached diagram are schematics of the representative processes which may be used for polypropylene manufacture.

In Fig. 6 a particular environment embodying the present invention is depicted. In Figure 6 a continuous process is shown, although the present invention is equally applicable to batch type operations. A stream of high purity propylene is fed through line 11 into reactor A where it is agitated with a diluent, added through line 12, such as xylene with a Ziegler catalyst comprising 3/1 $TiCl_3/AlCl_3$ and diethyl aluminum chloride in the diluent. The reaction product is carried to precipitator B via line 10 where a catalyst deactivator such as methanol is introduced through line 13 and agitated with reaction product to deactivate the catalyst. The mixture from precipitator B passes the deasher C via line 14 where the contacting is continued and hence to filter D through line 15. In filter D the solvent and a substantial portion of the catalyst are removed through line 16 while the filter cake is taken through line 17 to be reslurried with xylene and passed by line 18 into the neutralization tank E. The neutralizing solution of the present invention, i.e., the alcohol and alkali solution, methanol and Na methylate, enter tank E through line 19 and are agitated with the slurried polymer particles which then pass through line 20 to filter F. The xylene, methanol Na methylate and further catalyst residuals are removed as filtrate through line 21 and the deashed, neutralized polypropylene comes out as a moist powder through line 22, which powder may be water washed (not shown) and subsequent dried (not shown).

EXAMPLES

The following examples are given to illustrate the advantages of the present invention.

Example 1 (comparative example)

A lab test was conducted to determine the efficiency of the chemical adsorbent of the present invention. Helium, containing a 100 vppm $CO_2$ in helium, was passed through a cylindrical plastic tube, 125 mm in length and 17 mm in diameter with a bulbous end being 30 mm in diameter containing approximately 32 grams of Katalco 59-3 chemical adsorbent composed of a modified alumina, in spherical form, having a diameter within the range of 0.32 to 0.48 cm (1/8" to 3/16"), a surface area of 60 $m^2$/g, a pore volume of 0.3 mL/g, and a bulk density of 880.8 $Kg/m^3$ (55 lb./$ft.^3$). The $CO_2$ content of the feed, i.e. helium, and the effluent was determined using a gas chromatographic analytical system that included a methanator followed by a Flame Ionization Detector commonly used in the industry. It was found that the effluent contained ca.1 vppm of $CO_2$.

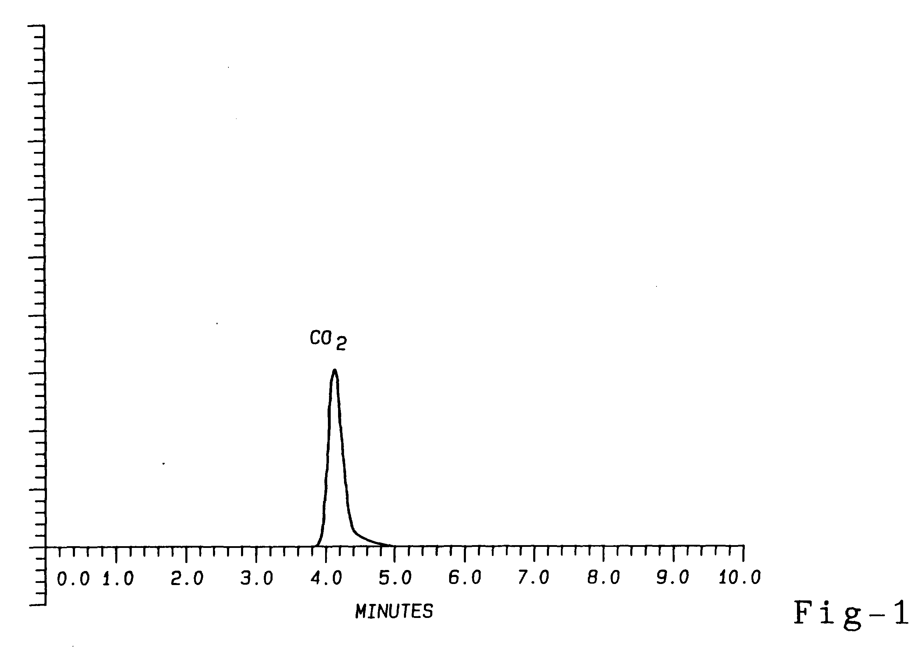

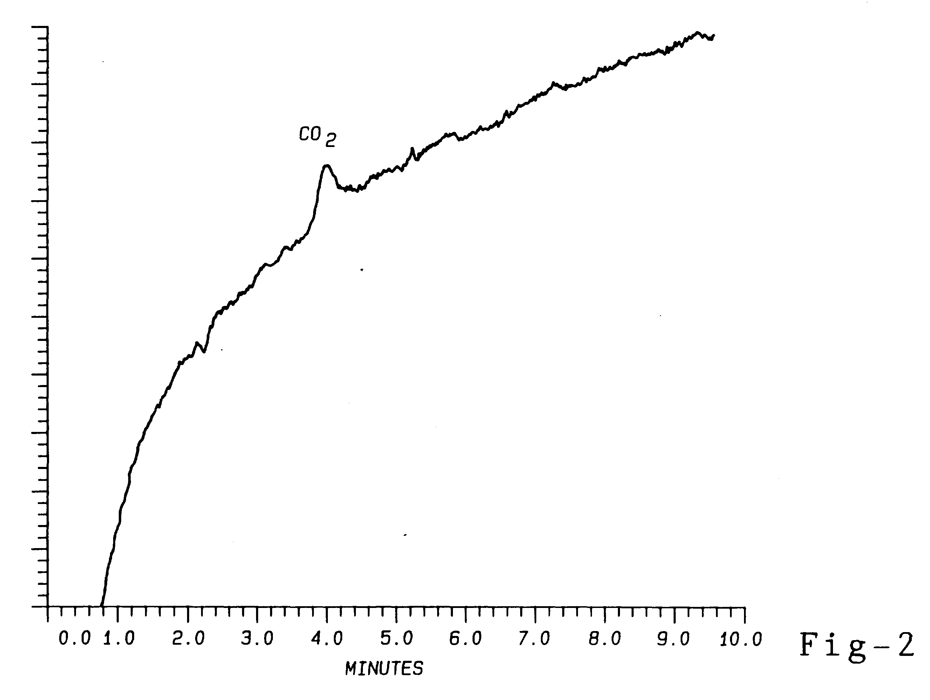

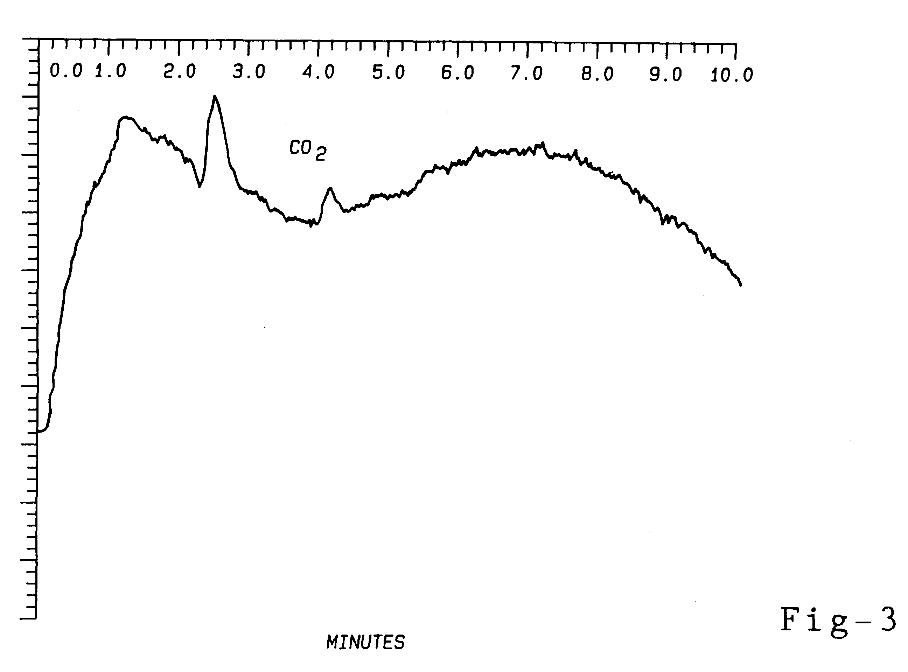

As shown in the attached Figures 1-3, the chemical adsorbent used in accordance with the present invention removed greater than 99.5% of the $CO_2$ in the He feed, wherein $CO_2$ in the feed was 145 mv signal; and the $CO_2$ in the effluent was 0.7 mv signal.

In accordance with the present invention, it has been discovered that the process for removing impurities from light ends using the chemical adsorbent in accordance with the present invention is valuable in basic as well as retrofit applications, particularly in high purity olefin manufacturing plants, such as propylene, ethylene and the like.

The high capacity and the sharp breakthrough curve, as illustrated in the attached figures, indicate that the chemical adsorbent used in accordance with the present invention improves the consistency of product quality even during upsets in the process upstream, thereby leading to an increase in impurity levels. Typical treatment techniques used for lowering impurity levels in propylene include absorption processes using an organic amine and/or caustic solutions followed by fractionation and drying using alumina driers.

However, such a system has a few disadvantages. In this regard, the impurities, such as $CO_2$, $H_2S$, and the like, are not lowered to "undetectable" levels. Also, in case of upsets in the absorption processed upstream, the impurity levels increase substantially and are beyond the capacity of the downstream processes to handle, thereby throwing the propylene product quality "off-spec".

In contrast, use of Katalco 59-3 in an adsorbent bed either "downstream of" or "in lieu of" the alumina driers offers several advantages. In accordance with the present invention, such use of Katalco 59-3 has been discovered to improve propylene quality by reducing certain trace impurities, such as $CO_2$ and $H_2S$, to extremely low levels. Also, it has been discovered that Katalco 59-3 has a significantly higher capacity to handle the higher impurity levels resulting from upsets in the upstream absorption processes, thereby contributing a significantly to the product quality assurance process.

In a typical high purity propylene operation embodying the process for removing impurities from hydrocarbon streams using the chemical adsorbent in accordance with the present invention, the chemical adsorbent can be loaded in adsorbent beds downstream from an alumina drier, or alternatively in lieu of them.

Example 2 (comparative example)

A gas consisting of 9.2 vppm $H_2S$ in helium was passed through the same tube as in Example 1 containing a new charge of Katalco 59-3. The $H_2S$ content of the feed and effluent was measured using a Tracor-Atlas Total Sulfur Analyzer. No detectable $H_2S$ was found in the effluent, i.e., <0.1 vppm.

Example 3 (comparative example)

Also, a gas consisting of 10.3 vppm COS in helium was passed through the same tube and Katalco 59-3 used in Experiment 2. The COS content of the feed and effluent was measured using a Tracor-Atlas Total Sulfur Analyzer. This experiment was performed on two separate days. On Day 1 the effluent was found to contain 4.3 vppm of COS and on Day 2 the effluent was found to contain 1.8 vppm COS.

Example 4

Also, a sample of high purity propylene was vaporized and passed through the same tube and Katalco 59-3 used in Experiment 3. The $AsH_3$ content of the feed and effluent was measured using an MDA Arsine Analyzer. The feed was found to contain 31 wppb $AsH_3$ and the effluent was found to contain 13 wppb $AsH_3$.

**Claims**

1. A process for removing $AsH_3$ or $PH_3$ impurities from hydrocarbon streams which comprises contacting a hydrocarbon stream containing an initial amount of $AsH_3$ or $PH_3$ impurity with a chemical adsorbent comprising a modified alumina having a surface area of from 10 to 300 $m^2/g$ and a pore volume of from 0.1 to 1 ml/g under contacting conditions effective to result in a purified hydrocarbon stream containing a reduced amount of impurities.

2. The process as defined by claim 1, wherein the modified alumina has a surface area of 60 $m^2/g$ and a pore volume of 0.3 ml/g.

3. The process as defined by any of the preceding claims, wherein the modified alumina has a bulk density within the range of from 640.6 to 960.9 Kg/$m^3$ (40 to 60 lb./ft.$^3$), preferably 880.8 Kg/$m^3$ (55 lb./ft.$^3$).

4. The process as defined by any of the preceding claims, wherein the modified alumina is spherical and has a diameter of from 0.15875 cm (1/16 in.) to 0.47625 cm (3/8 in.).

5. The process as defined by any of the preceding claims, wherein the modified alumina is impregnated with lithium, sodium, potassium, calcium, magnesium, or barium metal.

6. The process as defined by any of the preceding claims, wherein the modified alumina comprises alpha alumina or gamma alumina.

7. The process as defined by any of the preceding claims, wherein the contacting conditions comprise a temperature of from -12.2 to 65.6°C (10 to 150°F), preferably -1.1 to 48.9° C (30 to 120°F).

8. The process as defined by any of the preceding claims, wherein the contacting conditions comprise a pressure of from 344.758 to 3447.5 kPag (50 to 500 psig), preferably 1034.25 to 3413.25 kPag (150 to 350 psig).

9. The process as defined by any of the preceding claims, wherein the contacting conditions comprise a space velocity of from 0.1 to 25 VHSV, preferably 0.5 to 1 VHSV.

10. The process as defined by any of the preceding claims, wherein the hydrocarbon stream is in the liquid phase.

11. The process as defined by any of the preceding claims, wherein the hydrocarbon stream comprises an olefin or a saturated hydrocarbon, preferably having a carbon number 2, 3, or 4.

12. The process as defined by any of the preceding claims, wherein the modified alumina is loaded in adsorbent beds downstream from alumina driers.

13. The process as defined by any of the preceding claims, wherein the impurities additionally comprise COS, $H_2O$, $CO_2$, or $H_2S$.

## Patentansprüche

1. Verfahren zur Entfernung von $AsH_3$- oder $PH_3$-Verunreinigungen aus Kohlenwasserstoffströmen, bei dem ein Kohlenwasserstoffstrom, der eine Anfangsmenge an $AsH_3$- oder $PH_3$-Verunreinigungen enthält, mit einem chemischen Adsorbens, das modifiziertes Aluminiumoxid mit einer Oberfläche von 10 bis 300 $m^2$/g und einem Porenvolumen von 0,1 bis 1 ml/g umfaßt, unter Kontaktbedingungen in Kontakt gebracht wird, die wirksam sind, um zu einem gereinigten Kohlenwasserstoffstrom zu führen, der eine verringerte Menge Verunreinigungen enthält.

2. Verfahren nach Anspruch 1, bei dem das modifizierte Aluminiumoxid eine Oberfläche von 60 $m^2$/g und ein Porenvolumen von 0,3 ml/g aufweist.

3. Verfahren nach einem der vorhergehenden Ansprüche, bei dem das modifizierte Aluminiumoxid eine Schüttdichte im Bereich von 640,6 bis 960,9 kg/$m^3$ (40 bis 60 lb/$ft^3$), vorzugsweise 880,8 kg/$m^3$ (55 lb/$ft^3$) aufweist.

4. Verfahren nach einem der vorhergehenden Ansprüche, bei dem das modifizierte Aluminiumoxid kugelförmig ist und einen Durchmesser von 0,15875 cm (1/16 Inch) bis 0,47625 cm (3/8 Inch) aufweist.

5. Verfahren nach einem der vorhergehenden Ansprüche, bei dem das modifizierte Aluminiumoxid mit Lithium-, Natrium-, Kalium-, Calcium-, Magnesium- oder Bariummetall imprägniert ist.

6. Verfahren nach einem der vorhergehenden Ansprüche, bei dem das modifizierte Aluminiumoxid α-Aluminiumoxid oder γ-Aluminiumoxid umfaßt.

7. Verfahren nach einem der vorhergehenden Ansprüche, bei dem die Kontaktbedingungen eine Temperatur von -12,2 bis 65,6 °C (10 bis 150 °F), vorzugsweise -1,1 bis 48,9 °C (30 bis 120 °F) umfassen.

8. Verfahren nach einem der vorhergehenden Ansprüche, bei dem die Kontaktbedingungen einen Überdruck von 344,758 bis 3447,5 kPa (50 bis 500 psig), vorzugsweise 1034,25 bis 3413,25 kPa (150 bis 350 psig) umfassen.

9. Verfahren nach einem der vorhergehenden Ansprüche, bei dem die Kontaktbedingungen eine Raumgeschwindigkeit von 0,1 bis 25 VHSV, vorzugsweise 0,5 bis 1 VHSV umfassen.

10. Verfahren nach einem der vorhergehenden Ansprüche, bei dem der Kohlenwasserstoffstrom in der flüssigen Phase vorliegt.

11. Verfahren nach einem der vorhergehenden Ansprüche, bei dem der Kohlenwasserstoffstrom ein Olefin oder einen gesättigten Kohlenwasserstoff, vorzugsweise mit einer Kohlenstoffzahl von 2, 3 oder 4 umfaßt.

12. Verfahren nach einem der vorhergehenden Ansprüche, bei dem das modifizierte Aluminiumoxid in Adsorbensbetten stromabwärts von Aluminiumoxidtrocknern gefüllt ist.

13. Verfahren nach einem der vorhergehenden Ansprüche, bei dem die Verunreinigungen außerdem COS, $H_2O$, $CO_2$ oder $H_2S$ umfassen.

## Revendications

1. Procédé pour éliminer des impuretés consistant en $AsH_3$ ou $PH_3$ de courants d'hydrocarbures, qui comprend la mise en contact d'un courant d'hydrocarbures contenant une quantité initiale d'impuretés consistant en $AsH_3$ ou $PH_3$ avec un adsorbant chimique comprenant une amine modifiée ayant une surface spécifique de 10 à 300 m$^2$/g et un volume de pores de 0,1 à 1 ml/g dans des conditions de mise en contact efficaces pour qu'il en résulte un courant d'hydrocarbures purifié contenant une quantité réduite d'impuretés.

2. Procédé répondant à la définition suivant la revendication 1, dans lequel l'alumine modifiée a une surface spécifique de 60 m$^2$/g et un volume des pores de 0,3 ml/g.

3. Procédé répondant à la définition suivant l'une quelconque des revendications précédentes, dans lequel l'alumine modifiée a une masse volumique apparente comprise dans l'intervalle de 640,6 à 960,9 kg/m$^3$ (40 à 60 lb/ft$^3$), de préférence égale à 880,8 kg/m$^3$ (55 lb/ft$^3$).

4. Procédé répondant à la définition suivant l'une quelconque des revendications précédentes, dans lequel l'alumine modifiée est sphérique et a un diamètre de 0,15875 cm (1/16 in.) à 0,47625 cm (3/8 in.).

5. Procédé répondant à la définition suivant l'une quelconque des revendications précédentes, dans lequel l'alumine modifiée est imprégnée avec du lithium, du sodium, du potassium, du calcium, du magnésium ou du baryum métallique.

6. Procédé répondant à la définition suivant l'une quelconque des revendications précédentes, dans lequel l'alumine modifiée comprend l'alpha-alumine ou la gamma-alumine.

7. Procédé répondant à la définition suivant l'une quelconque des revendications précédentes, dans lequel les conditions de mise en contact comprennent une température de -12,2 à 65,6°C (10 à 150°F), de préférence de -1,1 à 48,9°C (30 à 120°F).

8. Procédé répondant à la définition suivant l'une quelconque des revendications précédentes, dans lequel les conditions de mise en contact comprennent une pression de 344,758 à 3447,5 kPa au manomètre (50 à 500 psi), de préférence de 1034,25 à 3413,25 kPa au manomètre (150 à 350 psi).

9. Procédé répondant à la définition suivant l'une quelconque des revendications précédentes, dans lequel les conditions de mise en contact comprennent une vitesse spatiale VSHV de 0,1 à 25, de préférence une VSHV de 0,5 à 1.

10. Procédé répondant à la définition suivant l'une quelconque des revendications précédentes, dans lequel le courant d'hydrocarbures est en phase liquide.

11. Procédé répondant à la définition suivant l'une quelconque des revendications précédentes, dans lequel le courant d'hydrocarbures comprend une oléfine ou un hydrocarbure saturé, ayant de préférence un nombre d'atomes de carbone égal à 2, 3 ou 4.

12. Procédé répondant à la définition suivant l'une quelconque des revendications précédentes, dans lequel l'alumine modifiée est chargée dans des lits d'adsorbant en aval des dessiccateurs d'alumine.

13. Procédé répondant à la définition suivant l'une quelconque des revendications précédentes, dans lequel les impuretés comprennent en outre COS, $H_2O$, $CO_2$ ou $H_2S$.

CO$_2$

0.0 1.0   2.0   3.0   4.0   5.0   6.0   7.0   8.0   9.0   10.0

MINUTES

Fig-1

$CO_2$

MINUTES

Fig-2

EP 0 580 736 B1

Fig-3

MINUTES

Fig-4

Fig-5

Fig-6

CATALYST
DEACTIVATOR

SLURRY
MEDIUM

ALCOHOL
ALKALI

ALCOHOL,
ALKALI,
SLURRY MEDIUM,
CATALYST RESIDUAL

DILUENT
CATALYST

10

13

14

18

19

21

REACTOR
A

DEASHER
(PRECIPI-
TATOR)
B

DEASHER
C

FILTER
D

NEUTRAL-
IZATION
TANK
E

FILTER
F

11

12

16

17

15

20

22

α-OLEFIN

DILUENT,
CATALYST

DEASHED
NEUTRALIZED
POLYMER

EP 0 580 736 B1